# FASCICULE DE BREVET EUROPEEN

(11) **EP 1 978 874 B1**
(45) Date de publication et mention de la délivrance du brevet: **29.06.2011**
(21) Numéro de dépôt: 07712637.3
(22) Date de dépôt: 05.01.2007
(51) Int. Cl.: A61B 6/00

(54) **SYSTEME D'IMAGERIE MEDICALE**
MEDIZINISCHES BILDGEBUNGSSYSTEM
MEDICAL IMAGING SYSTEM

(30) Priorité: 06.01.2006 FR 0600129
(43) Date de publication de la demande: 15.10.2008
(73) Titulaire: DESIGNERS DEVELOPERS DISTRIBUTORS ASSOCIATES (D3A) MEDICAL SYSTEMS, 45000 ORLEANS (FR)
(72) Inventeur: DO-HUU, Jean-Paul, F-78720 Cernay la Ville (FR)
(74) Mandataire: Pontet, Bernard
(86) Numéro de dépôt international: PCT/FR2007/000011
(87) Numéro de publication internationale: WO 2007/077395

(56) Documents cités:
- DE-U1- 8 905 588
- US-A- 3 790 805
- US-A- 4 868 845
- US-A- 5 475 730
- US-A1- 2003 190 014
- US-B1- 6 302 581

## Description

La présente invention concerne un système d'imagerie médicale. Plus précisément elle concerne un système d'imagerie médicale mobile faisant intervenir des rayons X.

Le domaine de l'invention est le domaine médical. L'invention s'applique plus particulièrement au domaine de l'imagerie médicale de haute précision, par l'utilisation des rayons X, d'une extrémité du corps d'un patient telle qu'un poignet, une cheville, etc., ou d'une articulation d'un patient telle qu'un genou, une coude, etc.,

Actuellement, il existe une pluralité de systèmes et de dispositifs mobiles d'imagerie médicale. Parmi ces systèmes et dispositifs mobiles on peut citer les appareils dédiés aux mammographies ou à l'imagerie dentaire, des dispositifs et systèmes, dits « Mobiles chirurgicaux » dédiés à des applications d'imagerie médicale en cours d'intervention chirurgicale, et des dispositifs et systèmes, dits «Mobiles radiologiques », utilisés pour effectuer une imagerie médicale au lit d'un patient.

Cependant ces systèmes et dispositifs ont des inconvénients respectifs. Les appareils dédiés aux mammographies ou à l'imagerie dentaire ne sont pas utilisables pour une imagerie médicale d'une partie quelconque du corps d'un patient, et ne sont pas autonomes et ne permettent pas de couvrir toutes les opérations de la prise d'image médicale à la mise à disposition des outils de diagnostics. De plus, les appareils de mammographie ou d'imagerie dentaire ne permettent pas de couvrir certaines incidences de prise de vue tout en permettant une reproductibilité de ces incidences.

Les mobiles chirurgicaux présentent des problèmes d'encombrements car ils sont souvent composés de plusieurs ensembles. De plus, il ne sont pas pratiques à manipuler, et souffrent d'un manque de précision dans la reproduction des incidences de prise de d'image.

D'une manière similaire, les « mobiles radiologiques » ne sont pas équipés non plus de moyens permettant de couvrir toutes les opérations de la prise d'image médicale à la mise à disposition des outils de diagnostics. Par exemple, ils ne comprennent pas les moyens de traitement et de visualisation d'une image médicale permettant d'effectuer un diagnostic. Les « mobiles radiologiques » souffrent en plus d'un manque de précision de prise d'image. Le document US-A-5475730 décrit un appareil d'imagerie selon le préambule de la revendication 1.

Un objectif de l'invention est de palier aux inconvénients cités ci-dessus.

Un autre objectif de l'invention est de proposer un système d'imagerie médicale mobile permettant de couvrir un très grand nombre d'incidences de prise d'image et d'assurer la reproductibilité de ces incidences.

L'invention a aussi pour objectif de proposer un système d'imagerie médicale complet et autonome permettant d'effectuer toutes les opérations de la prise d'image médicale à la mise à disposition des outils de diagnostics.

L'invention vise aussi à proposer un système d'imagerie médicale permettant d'effectuer des images médicales de haute résolution.

L'invention a aussi pour objectif de proposer un système d'imagerie médicale plus performant et plus simple que les systèmes et dispositifs d'imagerie médicale faisant partie de l'état de l'art.

Enfin, un autre objectif de l'invention est de proposer un système d'imagerie médicale facile à fabriquer et peu coûteux. L'invention est décrite dans le jeu de revendications.

L'invention propose de remédier aux inconvénients précités par un système d'imagerie médicale comprenant une partie mobile composée d'un plan de travail, d'une potence supportant un bras équipé d'un émetteur de rayons et d'un récepteur de rayons, ladite partie mobile comprenant en outre :
- des moyens permettant audit bras de coulisser verticalement le long de la potence ; et
- des moyens de rotation de ladite potence autour d'un axe vertical ;

Avantageusement, le système selon l'invention comprend une partie mobile qui peut être déplacée. Les déplacements de cette partie mobile peuvent être effectués grâce à des moyens de moteurs qui peuvent être agencés de manière à ce qu'on puisse déplacer la partie mobile du système selon l'invention sans effectuer d'efforts physiques importants. Les déplacements de la partie mobile peuvent aussi être effectués au moyen de roues ou de moyens similaires équipant la partie mobile.

Le système selon l'invention est très pratique car il comprend une partie mobile qui peut être déplacée. Par conséquent, il est possible grâce au système selon l'invention de déplacer les moyens d'imagerie médicale jusqu'au patient et non l'inverse. Ceci permet de prendre des images médicales d'un patient qui ne peut pas être déplacé pour une raison quelconque. Le système comprenant un bras équipé d'un émetteur et d'un récepteur permet de prendre des images médicales avec une grande précision de prises d'images et permet de reproduire une pluralité d'incidences de prises d'image.

Avantageusement, le système selon l'invention peut s'adapter à une pluralité de positions de prises d'images. En effet, la liberté en rotation de la potence supportant l'émetteur et le récepteur d'ondes, rend possible une pluralité de position de prises d'images, car de cette manière il n'est plus nécessaire de déplacer tout le système d'imagerie médicale pour prendre deux images dans deux directions différentes : il suffit de tourner la potence supportant l'émetteur et le récepteur d'ondes.

De par sa composition, le système selon l'invention est avantageusement simple à produire, à mettre en oeuvre et à utiliser. De plus, les éléments composant le système selon l'invention sont eux-mêmes très simples et faciles à produire. En outre, le système selon l'invention peut être fabriqué avec des matériaux de faibles poids qui en diminuant le poids du système renforceront son côté pratique. Par ailleurs, le système selon l'invention est peu onéreux.

Dans une version avantageuse de l'invention, le système selon l'invention peut comprendre des moyens de rotation du bras autour d'un axe horizontal. Ces moyens de rotation peuvent être motorisés, et ainsi permettre la rotation du bras de manière totalement automatique et avec peu d'efforts physiques. De cette manière le système peut être doté d'une liberté en rotation de la potence autour d'un axe vertical et du bras autour d'un axe horizontal, le bras pouvant coulisser le long de la potence. Cette combinaison de rotations permet à l'ensemble émetteur et récepteur de pouvoir être positionné dans une région très vaste composée d'une multitude de positions permettant, par exemple, de prendre une image médicale d'une articulation et/ou d'une extrémité du corps d'un patient.

Le système selon l'invention peut comprendre des moyens permettant à au moins un élément composant le système d'être équilibré, de manière à ce qu'il puisse être déplacé plus facilement et rester dans une configuration choisie par l'utilisateur.

D'une manière avantageuse, le bras équipé d'un émetteur et d'un récepteur peut être agencé pour encadrer, horizontalement et près du sol, un support d'une partie du corps d'un patient. En effet, les libertés en rotation autour d'un axe vertical de la potence et éventuellement autour d'un axe horizontal du bras et la liberté du bras à coulisser le long de la potence permettent au bras d'être positionné horizontalement et près du sol et ainsi de pouvoir encadrer un support servant à supporter un membre d'un patient dont on veut prendre des images médicales.

Avantageusement, le système selon l'invention peut comprendre des moyens de stabilisation de la potence et/ou du bras dans une position quelconque. En effet, le bras est doté d'une liberté en translation le long de la potence qui, elle, est libre en rotation autour d'un axe vertical. De plus, le bras peut avantageusement être doté d'une liberté en rotation autour d'un axe horizontal. Le système selon l'invention peut comprendre des moyens de stabilisation d'au moins un élément composant le système selon l'invention. Ces moyens de stabilisation peuvent être des freins ou des moyens de type «vis pression », ou tout autre moyen équivalent.

D'une manière avantageuse, le plan de travail peut comporter une cavité dans laquelle peut s'engager le récepteur de manière à se poser verticalement sur ledit plan. Cette cavité permet d'ajuster le récepteur sur le plan de travail de façon à ce que le récepteur repose sur le plan de travail. Grâce à cette cavité, il est très pratique pour l'utilisateur de placer le bras équipé de l'émetteur et du récepteur dans une position verticale au dessus du plan de travail. Cette cavité permet de guider l'utilisateur dans le positionnement du récepteur sur le plan de travail. De plus, cette cavité permet au récepteur, quand il est engagé dans la cavité, d'être à la même hauteur que le plan de travail. Elle assure donc la continuité du plan de travail.

Dans une version avantageuse de l'invention, le récepteur peut être agencé de manière à ce qu'il est utilisé comme support d'au moins une partie d'un corps d'un patient. En effet, le bras portant le récepteur peut être placé verticalement au dessus du plan de travail. Dans cette position, le récepteur peut se poser verticalement sur le plan de travail ou éventuellement s'insérer dans une cavité présente sur le plan de travail. Le récepteur devient, dans cette configuration, une partie du plan de travail et peut être utilisé comme plan de travail. Il peut alors être utilisé comme support d'un membre ou d'une partie du corps d'un patient dont l'utilisateur veut prendre une image médicale.

Il est très avantageux que le plan de travail comprenne une cavité dans laquelle le récepteur peut s'engager, car le récepteur peut se poser sur le plan de travail de manière à ce que les faces supérieures du récepteur et du plan de travail soient dans un même plan. Ainsi, la continuité du plan de travail est conservée et le récepteur repose sur le plan de travail de manière à supporter le poids d'un membre ou d'une partie du corps d'un patient.

Avantageusement, le système selon l'invention peut être utilisé pour une imagerie médicale d'une cheville et/ou d'un poignet. En effet, le système selon l'invention peut se positionner de façon à effectuer une image médicale d'une cheville ou d'un poignet soit dans une position verticale du bras portant l'ensemble émetteur/récepteur d'ondes soit dans une position horizontale et près du sol de ce bras. La cheville ou le poignet peuvent être positionnés de manière à être posés sur le récepteur d'ondes. Ils peuvent aussi être posés sur un support qui sera ensuite encadré par l'ensemble émetteur d'ondes et récepteur d'ondes. La cheville ou le poignet peuvent aussi être amenés contre le récepteur en fonction d'une incidence choisie par l'utilisateur.

Plus avantageusement, le système selon l'invention peut être utilisé pour une imagerie médicale d'une articulation d'un corps d'un patient. En effet, le système selon l'invention peut être utilisé pour prendre une image médicale d'un genou, d'un coude, d'une hanche, etc. d'un patient. Il peut aussi être utilisé pour effectuer une image médicale d'une extrémité du corps d'un patient.

Avantageusement le système selon l'invention peut comprendre des moyens de protections d'au moins une partie du corps d'un patient des rayons émis par l'émetteur et/ou d'une collision avec au moins un élément du système. En effet, les rayons émis par les systèmes d'imageries médicales n'étant pas très bons pour la santé d'un individu, le système selon l'invention peut comprendre des moyens de protection d'une partie sensible du corps du patient contre ces ondes. D'une manière similaire, au moins un élément du système peut entrer en collision avec au moins une partie du corps d'un patient, lors du positionnement d'au moins un élément du système. Le système selon l'invention comprend des moyens de protection du patient contre ces risques de collision.

Avantageusement, au moins un élément du système peut comprendre des moyens de détection de contact. Par exemple, le récepteur peut comprendre des capteurs pour détecter un contact permettant au récepteur, d'une part, de se positionner d'une façon plus précise et, d'autre part, d'éviter les collisions avec un autre élément. Ces capteurs peuvent, par exemple, se trouver sur le contour du récepteur.

Dans une version avantageuse de l'invention, le système selon l'invention peut comprendre un écran orientable de visualisation d'une image médicale. Cet écran orientable peut être un écran électronique permettant la visualisation des images sous forme informatique.

Selon une particularité avantageuse de l'invention, le système selon l'invention peut comprendre des moyens informatiques de traitement d'une image médicale. Ces moyens informatiques peuvent comprendre un ordinateur équipé de moyens informatiques adéquats permettant le traitement d'images. De plus, la prise des images médicales peut être effectuée de manière informatique. En effet, le récepteur d'ondes peut être muni de capteurs numériques d'ondes. Ces capteurs d'ondes peuvent, lors d'une prise d'image envoyer des données vers des moyens informatiques grâce à des interfaces adéquates. Ces données peuvent ensuite être traitées par des moyens logiciels pour afficher l'image sur un écran orientable, par exemple. Les moyens de prise et de traitement d'image peuvent comprendre de multiples fonctionnalités de zoom, de prises d'images en rafale, etc.

Le système selon l'invention peut aussi comprendre des moyens d'impression d'une image telle qu'au moins une imprimante couleur ou non.

Dans une version avantageuse de l'invention le récepteur comprend au moins un capteur numérique permettant de visualiser un élément d'une taille inférieure ou égale à 200µm. D'une manière plus avantageuse le récepteur peut comprendre un capteur numérique permettant de visualiser des éléments de taille inférieure ou égale à 100µm.

Le capteur numérique peut être un capteur numérique de haute résolution spatiale présentant une résolution d'au moins 5 paires de lignes par mm et plus avantageusement d'au moins 8 paires de lignes par mm.

Dans une version avantageuse de l'invention, les rayons utilisés pour effectuer des images médicales sont des rayons X. Ainsi, le système peut comprendre un émetteur de rayon X et un récepteur de rayon X, muni de capteurs de rayons X.

Le système selon l'invention peut comprendre des moyens de motorisation permettant de positionner au moins un élément du système, avec peu d'efforts physiques. Le système peut aussi comprendre des moyens de commande de ces moyens de moteur.

Le système selon l'invention peut aussi comprendre des moyens de batterie. Ainsi, il sera possible avec le système selon l'invention de prendre des images médicales d'une partie du corps d'un patient situé à un endroit où il n'existe aucun accès à une source d'énergie ou en extérieur.

Avantageusement, le système selon l'invention peut comprendre une partie fixe comprenant au moins un support pouvant recevoir une partie du corps d'un patient et/ou de pouvoir reproduire une incidence de prise d'image. Le support peut être un support de forme sensiblement similaire à un « V », pouvant recevoir par exemple un pied pour prendre une image médicale d'une cheville. Il peut être de tout autre forme adapté à recevoir une partie ou un membre du corps d'un patient. La partie fixe du système peut avantageusement comprendre des moyens pour soutenir le patient et des moyens, tels que par exemple une chaise ou un lit, permettant au patient de se poser.

Dans une position avantageuse, le bras peut être vertical, l'émetteur de rayons se trouvant en haut du bras, et le récepteur de rayons peut reposer sur le plan de travail. Ainsi on peut prendre des images médicales d'un membre ou d'une partie du corps d'un patient, en posant ce membre sur le récepteur qui lui est posé sur le plan de travail.

Dans une position avantageuse, le bras peut être vertical, l'émetteur et le récepteur de rayons se trouvant à l'opposé du plan de travail. Ainsi, on peut effectuer une image médicale d'un membre d'un patient, sans utiliser le plan de travail. Ceci est particulièrement avantageux, quand on ne peut pas utiliser le plan de travail, ou quand le patient est posé sur un autre équipement tel qu'un lit ou une civière. Par exemple, si un patient est allongé sur un lit et qu'on ne peut pas le déplacer, on peut amener le système selon l'invention près du lit et poser le récepteur directement sur le lit du patient et ainsi effectuer une image médicale d'un de ses membres.

Enfin, dans une autre position avantageuse, le bras peut être horizontal, l'émetteur et le récepteur de rayons se trouvant à l'opposé du plan de travail. Cette position est particulièrement utile pour effectuer une image d'une partie d'un corps d'un patient situé près du sol. Ainsi, le récepteur et l'émetteur d'ondes peuvent être positionnés horizontalement et près du sol de manière à encadrer le membre du patient dont il faut faire des images. Dans cette configuration on peut, par exemple, prendre des images médicales d'une cheville d'un patient assis sur une chaise. Le pied du patient peut avantageusement être posé sur un support près du sol. Avantageusement, c'est le système qui vient s'adapter à la position du membre du patient et non l'inverse.

Le système selon l'invention est donc complet et autonome. Il peut comprendre des moyens permettant de réaliser toutes les opérations allant d'une prise d'image médicale jusqu'à la mise à disposition des outils de diagnostics.

D'autres avantages et caractéristiques apparaîtront à l'examen de la description détaillée d'un mode de réalisation nullement limitatif, et des dessins annexés sur lesquels
- les figures 1 à 2 sont des représentations simplifiées d'une partie mobile d'un système d'imagerie médicale selon l'invention, vu de côté dans deux configurations différentes ;
- la figure 3 est une représentation simplifiée d'une partie mobile d'un système d'imagerie médicale selon l'invention, vu de dessus ;
- la figure 4 est une représentation schématique d'une potence utilisée par un système d'imagerie médicale selon l'invention ;
- la figure 5 présente une partie mobile d'un système d'imagerie médicale selon l'invention ;
- la figure 6 représente une partie mobile d'un système d'imagerie médicale selon l'invention ; et
- les figures 7 à 10 représentent plusieurs dispositions d'une partie mobile d'un système d'imagerie médicale selon l'invention.

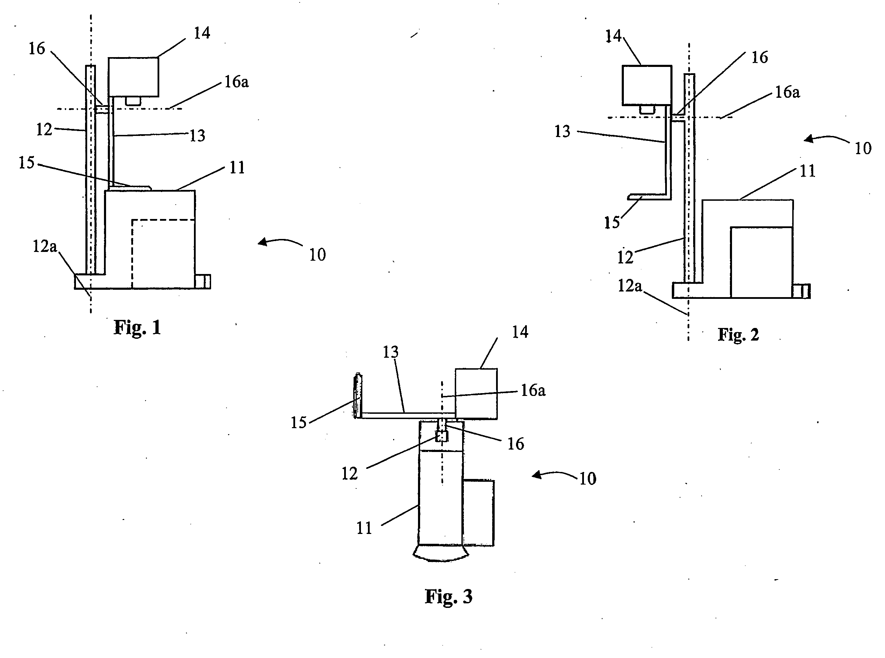

En référence aux figures 1 à 3, la partie mobile d'un système d'imagerie médicale comprend un plan de travail 11, une potence 12 supportant un bras 13 équipé d'un émetteur de rayons X 14 et un récepteur de rayons X 15. Le système comprend en outre des moyens de translation 16 du bras 13 le long de la potence 12.

La figure 1 représente la partie mobile 10 du système, de profil, dans une configuration où le bras 13 est positionné verticalement. Le récepteur 15 de rayons X repose sur le plan de travail 11. Sur la figure 2 la partie mobile du système est présentée de profil dans une configuration où le bras 13 équipé du récepteur 15 et de l'émetteur 14 est positionné verticalement à l'opposé du plan de travail 11. La figure 3, montre une vue de dessus de la partie mobile dans une configuration où le bras 13 est à l'opposé du plan de travail 11, dans une position horizontale. Pour passer de la configuration présentée en figure 2 à la configuration présentée en figure 3, le bras 13 effectue, grâce des moyens de rotation et translation 16, d'une part une rotation autour d'un axe horizontal passant par les moyens de rotation 16, d'autre part une translation le long de la potence 12. L'ordre des ces transformations n'est pas important.

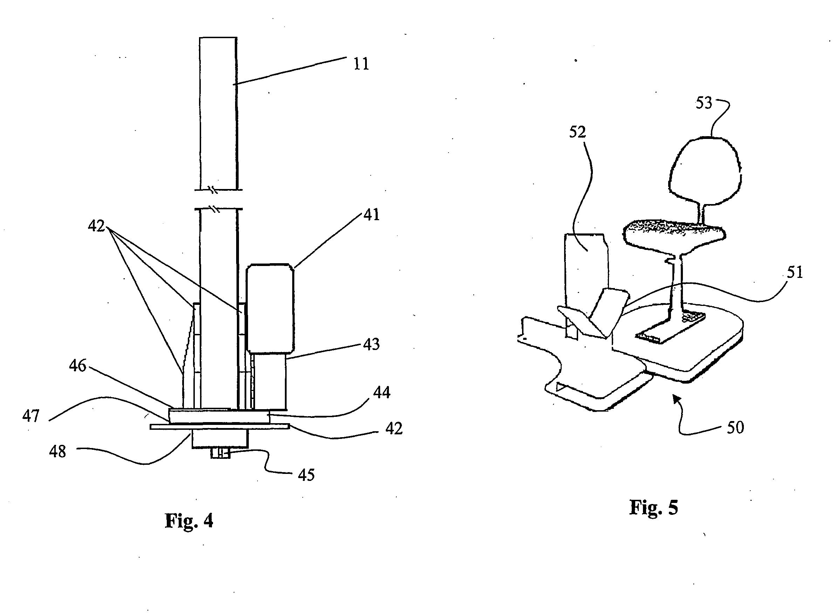

Pour passer de la configuration présentée sur la figure 1 à la configuration présentée en figure 2, la potence 12 supportant le bras 13, effectue une rotation autour d'un axe vertical passant par la potence. La figure 4 présente schématiquement les moyens permettant à cette potence d'effectuer cette rotation. Sur cette figure on peut apercevoir des moyens de motorisation 41 qui sont couplés à des moyens de réductions 43 pour atteindre une plus grade précision de positionnement de la potence 11 lors de sa rotation. La rotation est effectuéegrâce notamment à un plateau tournant 44, d'un disque d'encliquetage 46, d'une couronne de rotation 47 et d'un moyeu 48 soudé sur un châssis. L'ensemble représenté en figure 4 effectue une rotation autour d'un axe de rotation 45 et est assemblé et renforcé au moyen de tôles 42 soudées.

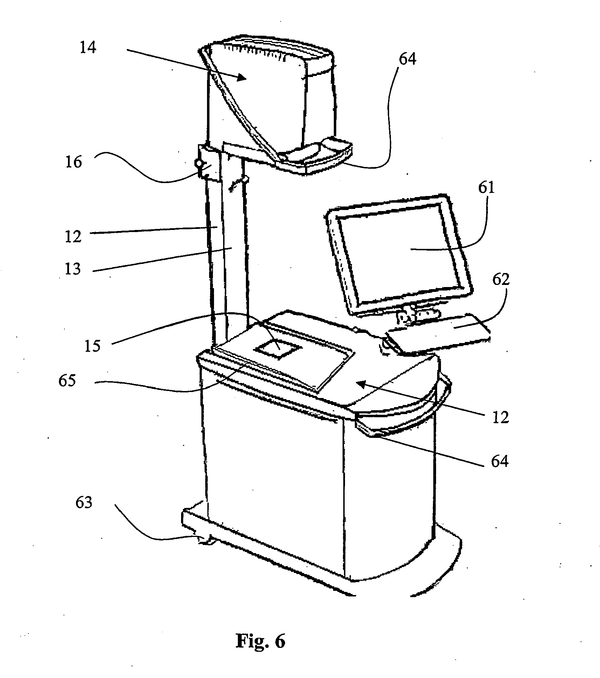

La figure 6 présente une partie mobile d'un système d'imagerie médicale selon l'invention. Sur cette figure il est possible d'apercevoir un plan de travail 11, une potence 12 supportant un bras 13 équipé d'un émetteur 14 de rayons X et d'un récepteur 15 de rayons X, des moyens 16 permettant au bras 13, d'une part de coulisser le long de la potence 12 et, d'autre part d'effectuer une rotation autour d'un axe horizontal. Sur cette figure on aperçoit aussi un écran 61 de visualisation des images médicales prises et de suivi des opérations effectuées, un clavier 62 permettant de saisir des informations relatives au patient et à aux images médicales prises ainsi que de contrôler les opérations effectuées. Cette partie mobile peut être déplacée grâce à des moyens de déplacements composés de roues 63. De plus, la partie mobile 10 ainsi que les différents éléments tels que le bras 13 et la potence 12 peuvent être déplacés au moyen de multiples prises 64. Le récepteur 15 peut s'engager dans une cavité 65 située sur le plan de travail de façon à se poser sur le plan de travail en respectant la continuité du plan de travail. Le patient peut alors poser un de ses membres sur le récepteur d'onde, car dans ce cas, le récepteur étant posé sur le plan de travail 11 peut supporter un poids plus important.

La figure 5 montre une partie fixe 50 du système d'imagerie médicale selon l'invention. Cette partie fixe 50 comprend un support 51 pour au moins une partie d'un corps d'un patient. Le support 51 est dans cet exemple de forme sensiblement similaire à un V de manière à ce qu'un patient puisse y poser un pied pour prendre par exemple des images d'une de ses chevilles. On peut aussi se servir de ce support pour effectuer toute sortes d'images médicales des membres inférieurs d'un patient. Le support 51 sert aussi à reproduire au moins une incidence de prise d'image. La partie fixe 50 du système peut comprendre des moyens, tels qu'une chaise ou un lit, permettant à un patient de se poser ou de se stabiliser pour la prise d'une image médicale. La partie fixe du système peut aussi comprendre des moyens de protection, tels qu'une plaque 52, d'au moins une partie du corps d'un patient, d'un opérateur ou d'une personne extérieure, contre les rayons intervenant dans la prise d'images médicales.

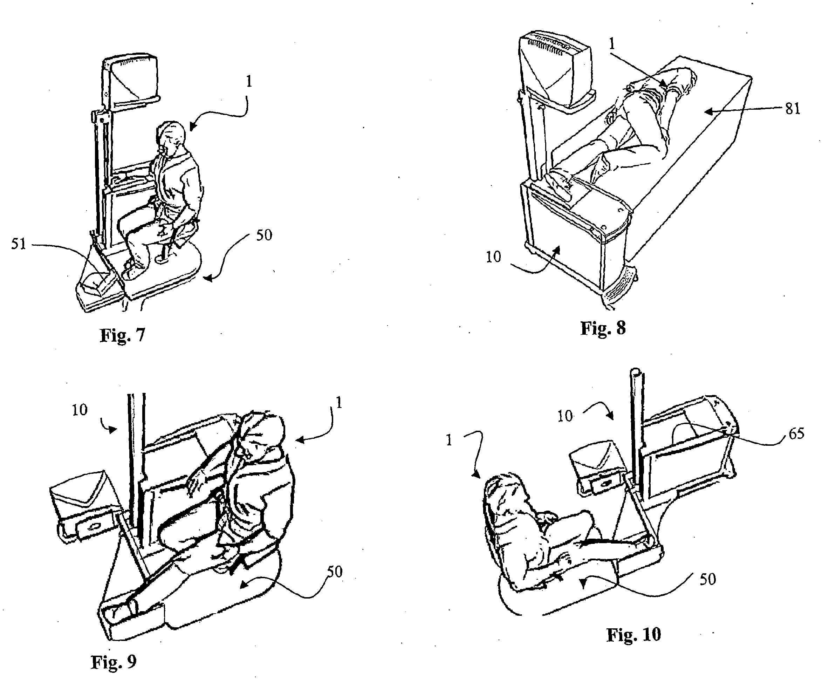

Les figures 7 à 10 montrent quatre configurations de prises d'image médicales avec le système selon l'invention. Ainsi, sur la figure 7 le patient est assis sur une chaise 53 fixée ou non sur la partie fixe 50 et pose son poignet sur le récepteur 15. Ce dernier est posé sur le plan de travail 11 au moyen de la cavité 65, le bras 13 est dans une position verticale. Sur la figure 8, le bras est toujours dans une position verticale permettant ainsi au récepteur 15 de se poser sur le plan de travail au moyen de la cavité 65. La partie mobile du système est accolée à un lit 81 sur lequel le patient est allongé. Cette configuration permet une prise d'image d'une cheville du patient sans que le patient soit déplacé. En effet, la partie mobile du système est déplacée et ajustée par rapport à la position du patient sans déranger le patient. D'où une très grande utilité du système selon l'invention. On peut aussi considérer que, dans une application particulière du système selon l'invention, le lit 81 fasse partie du système. Les figures 9 et 10 illustrent deux configurations de prises d'image médicale d'une cheville d'un patient. Le patient, assis sur la chaise 53, est soit du même côté que la partie mobile 10 du système, sur la figure 9, ou du côté opposé sur la figure 10. En effet, la partie fixe 50 et la partie mobile 10 du système sont prévues pour pouvoir être combinées qu'elles soient du même coté ou non et dans la même direction ou non. Sur les figures 9 et 10 le bras 13 équipé de l'émetteur 14 et du récepteur 15 est positionné horizontalement et près du sol. Dans cette position, l'émetteur 14 et le récepteur 15 entourent le support 51 sur lequel le patient pose son pied.

Quelque soit la configuration, le système est très peu encombrant. En effet, dans cet exemple, le système à les dimensions suivantes :
➢ Partie mobile
   - hauteur max. : 1882 mm ;
   - longueur max. : 1509mm ; et
   - largeur max. : 621 mm.
➢ Partie fixe
   - longueur max. : 1261mm ; et
   - largeur max. : 962 mm.

Dans la configuration la plus en encombrante, le système composé d'une partie mobile et d'une partie fixe ne dépasse pas les dimensions suivantes :
- hauteur max. : 1882 mm ;
- longueur max. : 2422 mm ; et
- largeur max. : 2070 mm.

La partie mobile du système peut fonctionner sans la partie fixe et peut être déplacé de manière pratique.

Le système selon l'invention n'est pas limité à l'exemple qui vient d'être décrit et peut prendre des formes variées et peut être appliqué à toutes sortes d'imageries médicales.

## Revendications

1. Système d'imagerie médicale comprenant une partie mobile (10) composée d'une potence (12) supportant un bras (13) équipé d'un émetteur de rayons (14) et d'un récepteur de rayons (15), ladite partie mobile (10) comprenant en outre :
- un plan de travail (11), et
- des moyens de rotation (41 à 48) de ladite potence (12) autour d'un axe vertical (12a) ;
**caractérisé en ce que** ladite partie mobile comprend en outre
- des moyens de translation (16) permettant audit bras (13) de coulisser verticalement le long de la potence (12) ;
- des moyens de rotation (16) du bras (13) autour d'un axe horizontal (16a),
lesdits moyens de rotation et de translation étant agencés de sorte que ledit bras est déplaçable entre :
- une première configuration dans laquelle ledit bras (13) est vertical, l'émetteur (14) de rayons se trouve en haut du bras (13), et le récepteur (15) de rayons repose sur le plan de travail (11), et
- une deuxième configuration dans laquelle ledit bras (13) est horizontal, l'émetteur (14) et le récepteur (15) de rayons se trouvant à l'opposé du plan de travail par rapport à la potence (12).

2. Système selon la revendication 1, **caractérisé en ce que** la potence (12) est de taille fixe et le bras (13) est à une distance fixe de la potence (12).

3. Système selon l'une quelconque des revendications précédentes, **caractérisé en ce qu'**il comprend en outre des moyens de stabilisation de la potence (12) et/ou du bras (13) et/ou de la partie mobile (10) dans une position quelconque.

4. Système selon l'une quelconque des revendications précédentes, **caractérisé en ce que** le plan de travail (11) comporte une cavité (65) dans laquelle peut s'engager le récepteur (15) de manière à se poser sur ledit plan (11).

5. Système selon la revendication 4, **caractérisé en ce que** le récepteur (15) est utilisé comme support d'au moins une partie d'un corps d'un patient.

6. Système selon l'une quelconque des revendications 1 à 4, **caractérisé en ce que** le bras (13) équipé d'un émetteur (14) et d'un récepteur (15) est agencé pour encadrer, horizontalement et près du sol, un support (51) d'une partie d'un corps d'un patient.

7. Système selon l'une quelconque des revendications précédentes, **caractérisé en ce qu'**il comprend en outre des moyens de protections (52) d'au moins une partie du corps d'un patient des rayons émis par l'émetteur (14) et/ou d'une collision avec au moins un élément du système.

8. Système selon l'une quelconque des revendications précédentes, **caractérisé en ce qu'**il comprend en outre un écran orientable (61) de visualisation d'une image médicale.

9. Système selon l'une quelconque des revendications précédentes, **caractérisé en ce qu'**il comprend en outre des moyens informatiques de traitement d'une image médicale.

10. Système selon l'une quelconque des revendications précédentes, **caractérisé en ce que** les rayons sont des rayons X.

11. Système selon l'une quelconque des revendications précédentes, **caractérisé en ce qu'**il comprend en outre des moyens de motorisation (41) permettant de positionner au moins un élément dudit système.

12. Système selon l'une quelconque des revendications précédentes, **caractérisé en ce que** le récepteur (15) comprend au moins un capteur numérique permettant de visualiser un élément d'une taille inférieure ou égale à 200µm.

13. Système selon l'une quelconque des revendications précédentes, **caractérisé en ce qu'**il comprend en outre une partie fixe (50) comprenant au moins un support (51) pouvant recevoir une partie du corps d'un patient et/ou de pouvoir reproduire une incidence de prise d'image.

14. Système selon l'une quelconque des revendications 1 à 13, **caractérisé en ce que** dans une position le bras (13) est vertical, l'émetteur (14) et le récepteur (15) de rayons se trouvant à l'opposé du plan de travail par rapport à la potence (12).

15. Utilisation du système selon l'une quelconque des revendications précédentes pour une imagerie médicale d'une cheville et/ou d'un poignet.

16. Utilisation du système selon l'une quelconque des revendications précédentes, pour une imagerie médicale d'une articulation d'un corps d'un patient.

## Claims

1. Medical imaging system comprising a mobile part (10) composed of a pillar stand (12) supporting an arm (13) equipped with a radiation emitter (14) and a radiation receiver (15), said mobile part (10) comprising moreover:
- a work surface (11), and
- means of rotation (41 to 48) of said pillar stand (12) about a vertical axis (12a);
**characterized in that** said mobile part also comprises:
- means of translation (16) for allowing said arm (13) to slide vertically along the pillar stand (12); and
- means of rotation (16) of the arm (13) about a horizontal axis (16a),
said means of rotation and means of translation being arranged so that said arm is moveable between:
- a first configuration in which said arm (13) is vertical, the radiation emitter (14) is at the top of the arm (13), and the radiation receiver (15) rests on the work surface (11), and
- a second configuration in which said arm (13) is horizontal, the radiation emitter (14) and the radiation receiver (15) being opposite the work surface in relation to the pillar stand (12).

2. System according to claim 1, **characterized in that** the pillar stand (12) is of a fixed size and the arm (13) is at a fixed distance from the pillar stand (12).

3. System according to any one of the previous claims, **characterized in that** it comprises moreover means for stabilizing the pillar stand (12) and/or the arm (13) and/or the mobile part (10) in any position.

4. System according to any one of the previous claims, **characterized in that** the work surface (11) comprises a cavity (65) in which the receiver (15) can be fitted in order to be fixed on said work surface (11).

5. System according to claim 4, **characterized in that** the receiver (15) is used as a support for at least one part of the body of a patient.

6. System according to any one of the claims 1 to 4, **characterized in that** the arm (13) equipped with an emitter (14) and a receiver (15) is arranged to frame, horizontally and close to the ground, a support (51) for one part of the body of a patient.

7. System according to any one of the previous claims, **characterized in that** it comprises moreover means of protection (52) of at least one part of the body of a patient from the radiation emitted by the emitter (14) and/or a collision with at least one element of the system.

8. System according to any one of the previous claims, **characterized in that** it comprises moreover an adjustable screen (61) for displaying a medical image.

9. System according to any one of the previous claims, **characterized in that** it comprises moreover computer means for processing a medical image.

10. System according to any one of the previous claims, **characterized in that** the radiation is X-rays.

11. System according to any one of the previous claims, **characterized in that** it comprises moreover means of motorization (41) making it possible to position at least one element of said system.

12. System according to any one of the previous claims, **characterized in that** the receiver (15) comprises at least one digital sensor making it possible to visualize an element of a size less than or equal to 200 µm.

13. System according to any one of the previous claims, **characterized in that** it comprises moreover a fixed part (50) comprising at least one support (51) which can receive a part of the body of a patient and/or reproduce an imaging view.

14. System according to any one of claims 1 to 13, **characterized in that** in one position the arm (13) is vertical, the radiation emitter (14) and the radiation receiver (15) being opposite the work surface in relation to the pillar stand (12).

15. Use of the system according to any one of the previous claims, for the medical imaging of an ankle and/or a wrist.

16. Use of the system according to any one of the previous claims, for the medical imaging of a joint of the body of a patient.

## Patentansprüche

1. Medizinisches Bildgebungssystem umfassend ein bewegliches Teil (10) zusammengesetzt aus einer Stütze (12), welche einen Arm (13) trägt, der mit einem Sender (14) für Strahlen und einem Empfänger (15) für Strahlen ausgerüstet ist, das bewegliche Teil (10) weiter umfassend:
- eine Arbeitsfläche (11), und
- Drehmittel (41 bis 48) zur Rotation der Stütze (12) um eine vertikale Achse (12a);
**dadurch gekennzeichnet, dass** das bewegliche Teil weiter umfasst:
- Translationsmittel (16) zur Verschiebung des Arms (13) in vertikaler Richtung entlang der Stütze (12);
- Drehmittel (16) zur Rotation des Arms (13) um eine horizontale Achse (16a),
wobei die Drehmittel und die Translationsmittel derart angeordnet sind, dass der Arm verstellbar ist, zwischen:
- einer ersten Stellung in welcher der Arm (13) vertikal ist, der Sender (14) für Strahlen sich oberseitig am Arm (13) befindet und der Empfänger (15) für Strahlen auf der Arbeitsfläche (11) liegt, und
- einer zweiten Stellung in welcher der Arm (13) horizontal ist, der Sender (14) für Strahlen und der Empfänger (15) für Strahlen sich gegenüber der Arbeitsfläche in Bezug auf die Stütze (12) befinden.

2. System nach Anspruch 1, **dadurch gekennzeichnet, dass** die Stütze (12) eine festgesetzte Grösse aufweist und dass der Arm (13) einen festgesetzten Abstand von der Stütze (12) aufweist.

3. System nach einem der vorangehenden Ansprüche, **dadurch gekennzeichnet, dass** es weiter Mittel zur Stabilisierung der Stütze (12) und/oder des Arms (13) und/oder des beweglichen Teils (10) in einer beliebigen Position umfasst.

4. System nach einem der vorangehenden Ansprüche, **dadurch gekennzeichnet, dass** die Arbeitsfläche (11) eine Vertiefung (65) aufweist, in welche der Empfänger (15) derart eingreifen kann, dass er auf der besagten Fläche (11) steht.

5. System nach Anspruch 4, **dadurch gekennzeichnet, dass** der Empfänger (15) als Stütze für mindestens einen Teil eines Körpers eines Patienten vorgesehen ist.

6. System nach einem der Ansprüche 1 bis 4, **dadurch gekennzeichnet, dass** der Arm (13), welcher mit einem Sender (14) und einem Empfänger (15) versehen ist, derart angeordnet ist, dass er horizontal und in der Nähe des Bodens eine Auflage (51) für einen Teil eines Körpers eines Patienten beschränkt.

7. System nach einem der vorangehenden Ansprüche, **dadurch gekennzeichnet, dass** es weiter Mittel zum Schutz (52) umfasst, welche wenigstens einen Teil eines Körpers eines Patienten vor vom Sender (14) emittierte Strahlen und/oder vor einer Kollision mit wenigstens einem Element des Systems schützen.

8. System nach einem der vorangehenden Ansprüche, **dadurch gekennzeichnet, dass** es weiter einen verstellbaren Bildschirm (61) zur Anzeige eines medizinischen Bilds umfasst.

9. System nach einem der vorangehenden Ansprüche, **dadurch gekennzeichnet, dass** es weiter Mittel zur elektronischen Datenverarbeitung zur Verarbeitung eines medizinischen Bilds umfasst.

10. System nach einem der vorangehenden Ansprüche, **dadurch gekennzeichnet, dass** die Strahlen Röntgenstrahlen sind.

11. System nach einem der vorangehenden Ansprüche, **dadurch gekennzeichnet, dass** es weiter Antriebsmittel (41) zur Positionierung von mindestens einem Element des Systems umfasst.

12. System nach einem der vorangehenden Ansprüche, **dadurch gekennzeichnet, dass** der Empfänger (15) mindestens einen digitalen Sensor zur Visualisierung eines Elements von einer Grösse kleiner als oder gleich 200 µm umfasst.

13. System nach einem der vorangehenden Ansprüche, **dadurch gekennzeichnet, dass** es weiter einen feststehenden Teil (50) umfasst, umfassend mindestens eine Auflage (51) zur Aufnahme eines Teils eines Körpers eines Patienten und/oder zur Nachbildung des Einfallswinkels der Aufnahme des Bildes.

14. System nach einem der Ansprüche 1 bis 13, **dadurch gekennzeichnet, dass** in einer Stellung der Arm (23) vertikal ist, der Sender (14) für Strahlen und der Empfänger (15) für Strahlen sich in Bezug auf die Stütze (12) gegenüber der Arbeitsfläche befinden.

15. Verwendung des Systems nach einem der vorangehenden Ansprüche für eine medizinische Bildgebung eines Knöchels und/oder eines Handgelenks.

16. Verwendung des Systems nach einem der vorangehenden Ansprüche für eine medizinische Bildgebung eines Gelenks eines Köpers eines Patienten.
